# EUROPEAN PATENT SPECIFICATION

(11) **EP 1 255 537 B1**
(45) Date of publication and mention of the grant of the patent: **19.04.2006**
(21) Application number: 01905717.3
(22) Date of filing: 01.02.2001
(51) Int. Cl.: A61K 31/00, A61K 31/4709, A61P 35/00, A61P 35/04, A61K 31/4745, A61K 31/517, A61K 31/519

(54) **FARNESYL PROTEIN TRANSFERASE INHIBITORS FOR TREATING BREAST CANCER**
FARNESYL PROTEIN TRANSFERASE INHIBITOREN ZUR BEHANDLUNG VON BRUSTKREBS
INHIBITEURS DE LA FARNESYL PROTEINE TRANSFERASE POUR LE TRAITEMENT DU CANCER DU SEIN

(30) Priority: 04.02.2000 EP 00200373
(43) Date of publication of application: 13.11.2002
(73) Proprietor: JANSSEN PHARMACEUTICA N.V., 2340 Beerse (BE)
(72) Inventor: PALMER, Peter Albert, Janssen Pharmaceutica N.V, B-2340 Beerse (BE); HORAK, Ivan, David, Yardley, PA 19067 (US)
(74) Representative: Daelemans, Frank F.R.
(86) International application number: PCT/EP2001/001032
(87) International publication number: WO 2001/056552

(56) References cited:
- WO-A-00/01411
- WO-A-00/12498
- WO-A-01/45740
- WO-A-01/62234
- WO-A-01/64252
- WO-A-97/21701
- ZUJEWSKI J (REPRINT) ET AL: "Phase I and pharmacokinetic study of farnesyl protein transferase inhibitor R115777 in advanced cancer" JOURNAL OF CLINICAL ONCOLOGY, (FEB 2000) VOL. 18, NO. 4, PP. 927-941. PUBLISHER: LIPPINCOTT WILLIAMS & WILKINS, 530 WALNUT ST, PHILADELPHIA, PA 19106-3621. ISSN: 0732-183X., XP000957509 NATL CANC INST, DIV CLIN SCI, MED BRANCH, 9000 ROCKVILLE PIKE, BETHESDA, MD 20892 (Reprint);NATL INST HLTH, CTR CLIN, BETHESDA, MD; NEI, BETHESDA, MD 20892; SAIC FREDERICK, FREDERICK, MD; JANSSEN RES INST, TITUSVILLE, NJ; JANSSEN RES FDN, B-2340 BEERSE, BELGIUM; ORTHOCLIN DIAGNOST, ROCHESTER, NY; JO
- ZUJEWSKI, J. ET AL: "Phase I trial of farnesyl-transferase inhibitor, R115777, in advanced cancer." PROCEEDINGS OF THE AMERICAN ASSOCIATION FOR CANCER RESEARCH ANNUAL MEETING, (MARCH, 1998) VOL. 39, PP. 270. MEETING INFO.: 89TH ANNUAL MEETING OF THE AMERICAN ASSOCIATION FOR CANCER RESEARCH NEW ORLEANS, LOUISIANA, USA MARCH 28-APRIL 1, 1998 AMERICAN, XP000952726
- PUSZTAI L. ET AL: "Chemo-signal therapy, an emerging new approach to modify drug resistance in breast cancer." CANCER TREATMENT REVIEWS, (1999) 25/5 (271-277)., XP000956202
- VIGUSHIN D M (REPRINT) ET AL: "Phase I and pharmacokinetic study of D-limonene in patients with advanced cancer" CANCER CHEMOTHERAPY AND PHARMACOLOGY, (JUL 1998) VOL. 42, NO. 2, PP. 111-117. PUBLISHER: SPRINGER VERLAG, 175 FIFTH AVE, NEW YORK, NY 10010. ISSN: 0344-5704., XP000957508 CHARING CROSS HOSP, DEPT MED ONCOL, CANC RES CAMPAIGN LABS, FULHAM PALACE RD, LONDON W6 8RF, ENGLAND (Reprint);INST CANC RES, CANC RES CAMPAIGN, CTR CANC THERAPEUT, SUTTON SM2 5NG, SURREY, ENGLAND; UNIV NEWCASTLE UPON TYNE, SCH MED, CANC RES UNIT, NEWCASTLE TYNE NE2 4HH, TYNE & WEAR, ENGLAND; UNIV S
- GELB, MICHAEL H. ET AL: "The inhibition of protein prenyltransferases by oxygenated metabolites of limonene and perillyl alcohol" CANCER LETT. (SHANNON, IREL.) (1995), 91(2), 169-75, XP000956493
- PUNT, C.J.A. (1) ET AL: "Phase I trial with farnesyltransferase inhibitor R115777 in patients (pts) with advanced solid tumors." EUROPEAN JOURNAL OF CANCER, (SEPT., 1999) VOL. 35, NO. SUPPL. 4, PP. S291. MEETING INFO.: ECCO 10: THE EUROPEAN CANCER CONFERENCE VIENNA, AUSTRIA SEPTEMBER 12-16, 1999 FEDERATION OF EUROPEAN CANCER SOCIETIES., XP000957507
- SCHELLENS, J. H. M. (1) ET AL: "Phase I and pharmacologic study with the novel farnesyltransferase inhibitor (FTI) R115777." PROCEEDINGS OF THE AMERICAN ASSOCIATION FOR CANCER RESEARCH ANNUAL MEETING, (MARCH, 1999) VOL. 40, PP. 724. MEETING INFO.: 90TH ANNUAL MEETING OF THE AMERICAN ASSOCIATION FOR CANCER RESEARCH PHILADELPHIA, PENNSYLVANIA, USA APRIL 10-14, 1999 AMERICAN, XP000952727
- SKRZAT, S. (1) ET AL: "R115777, a novel imidazole farnesyl protein transferase inhibitor (FTI) with potent oral antitumor activity." PROCEEDINGS OF THE AMERICAN ASSOCIATION FOR CANCER RESEARCH ANNUAL MEETING, (MARCH, 1998) VOL. 39, PP. 317-318. MEETING INFO.: 89TH ANNUAL MEETING OF THE AMERICAN ASSOCIATION FOR CANCER RESEARCH NEW ORLEANS, LOUISIANA, USA MARCH 28-APRIL 1, 1998 AMER, XP000952725
- HEAD J. ET AL BREAST CANCER RESEARCH vol. 6, no. 6, 2004, pages 262 - 268
- PRENDERGAST G.C. ET AL MOL CELL BIOLOGY vol. 14, 1994, pages 4193 - 4202
- KELLAND L.R. ET AL CLINICAL CANCER RESEARCH vol. 7, 2001, pages 3544 - 3550
- JOHNSTON S.R. ET AL JOURNAL OF CLINICAL ONCOLOGY vol. 21, no. 13, 2003, pages 2492 - 2499
- WORLD HEALTH ORGANIZATION OFFSET PUBLICATION, no. 48, 1979, Geneva
- ROWINSKY ET AL JOURNAL OF CLINICAL ONCOLOGY vol. 17, no. 11, 1999, pages 3631 - 3652

## Description

The present invention is concerned with the finding that a farnesyl protein transferase inhibitor is useful for preparing a pharmaceutical composition for treating advanced breast cancer.

Oncogenes frequently encode protein components of signal transduction pathways which lead to stimulation of cell growth and mitogenesis. Oncogene expression in cultured cells leads to cellular transformation, characterized by the ability of cells to grow in soft agar and the growth of cells as dense foci lacking the contact inhibition exhibited by non-transformed cells. Mutation and/or overexpression of certain oncogenes is frequently associated with human cancer. A particular group of oncogenes is known as *ras* which have been identified in mammals, birds, insects, mollusks, plants, fungi and yeasts. The family of mammalian *ras* oncogenes consists of three major members ("isoforms") : H-*ras,* K-*ras* and N-*ras* oncogenes. These *ras* oncogenes code for highly related proteins generically known as p21^{*ras*}. Once attached to plasma membranes, the mutant or oncogenic forms of p21^{*ras*} will provide a signal for the transformation and uncontrolled growth of malignant tumor cells. To acquire this transforming potential, the precursor of the p21^{*ras*} oncoprotein must undergo an enzymatically catalyzed famesylation of the cysteine residue located in a carboxyl-terminal tetrapeptide. Therefore, inhibitors of the enzyme that catalyzes this modification, farnesyl protein transferase, will prevent the membrane attachment of p21^{*ras*} and block the aberrant growth of *ras*-transformed tumors. Hence, it is generally accepted in the art that farnesyl transferase inhibitors can be very useful as anticancer agents for tumors in which *ras* contributes to transformation.

It has been estimated that as many as 30-40% of human tumours may contain a ras mutation, with some tumours, such as colon and lung, showing ras mutations in around 50% and 90% of tumours, respectively. K-ras and Ha-ras mutations have been identified in breast cancer tumours, but at low levels (approximately 5%).

However, although ras mutations are relatively infrequent in breast cancer, there is evidence to suggest that the pathways which ras services may still be deregulated in breast cancer cells (Clark GJ and Der CJ, Breast Cancer Res. Treat. 1995, 35 (1), 133-144). Recent identification of many of the components of the ras signal transduction pathway has defined a network of protooncogene proteins controlling diverse signalling events that regulate cell growth and differentiation. Mutations that alter the function of any one component of this signal pathway may trigger the same oncogenic events as a mutation of ras itself. Moreover, ras-related proteins, such as TC21/R-Ras2, have been shown to possess the ability to trigger malignant transformation in MCF-10A human breast epithelial cells lines via signalling pathways shared with ras proteins (Clark G.J. *et al ,* Oncogene, 1996, 12(1), 169-76). Also, TC21 protein expression was found to be greatly elevated in 7 of 9 breast tumour lines when compared to untransformed MCF-10A cells.

Bland *et al* (Ann. Surg. 1995, 221(6), 706-18) looked at oncogene protein expression as prognostic discriminants for breast cancer. Of the individual oncogenes examined (c-fos, c-myc, Ha-ras and p53), the presence of Ha-ras and c-fos gave the greatest prediction for poor survival.

Breast cancer is the most common female malignancy and the main cause of death from cancer in women. Each year approximately 30,000 new cases are diagnosed and there are nearly 16,000 deaths in the UK; about 1 in 12 women will develop breast cancer at some time in their life.

Patients with clinically evident distant metastases are still incurable, although the disease may be controlled for periods longer than 5 or 10 years in some patients. However the median survival of all patients with metastatic disease is approximately 2 to 3 years, and the search for additional effective therapies continues.

Currently patients with indolent disease, i.e. disease characterised by the presence of bone, soft tissue or non-life threatening visceral metastases are generally treated in the first instance with an endocrine therapy , such as an aromatase inhibitor, anti-oestrogen or progestogen. Depending on their response to that therapy, patients may receive further endocrine treatment before being considered for chemotherapy. Patients with aggressive disease, characterised by widespread symptomatic metastases or extensive visceral involvement, will normally be considered for combination therapy, such as FEC or CAF, as a more rapid response is desirable in these patients. Patients who have oestrogen receptor (ER) negative tumours may also be treated in the first instance with chemotherapy, since hormonal therapy is largely ineffective in this group.

WO-97/21701 describes the preparation, formulation and pharmaceutical properties of certain farnesyl protein transferase inhibiting (imidazoly-5-yl)methyl-2-quinolinones.

Proceedings of the American Association for Cancer Research, Vol. 39, March 1998, p. 270 describes phase I trial of the farnesyl-transferase inhibitor, R115777, in advanced cancer.

Unexpectedly, we have now found that a farnesyl protein transferase inhibitor falling within the scope of the above WO-97/21701 and identified therein as compound 75, referred to below, has clinical activity in advanced breast cancer. This effect is especially surprising as treatment with the farnesyl protein transferase inhibitor results in shrinkage of the tumor rather than simply delaying tumor progression. This effect is in contrast to the suggestion in Rowinsky et al, Journal of Clinical Oncology, Vol 17, No. 11 (November), 1999, pages 3631-3652 at page 3646 that tumor growth inhibition or "cytostasis" may be the principal therapeutic effect of FTase inhibitors.

The present invention is concerned with the use of a farnesyl protein transferase inhibitor for the preparation of a pharmaceutical composition for treating advanced breast cancer, wherein said farnesyl protein transferase inhibitor is (+)-6-[amino(4-chlorophenyl)(1-methyl-1*H*-imidazol-5-yl)methyl]-4-(3-chloro-phenyl)-1-methyl-2(1*H*)-quinolinone; or a pharmaceutically acceptable acid addition salt or stereochemically isomeric form thereof.

The term "advanced breast cancer" is used herein to denote breast cancer which has not responded to previous treatment, or which has recurred following such treatment, and also breast cancer in patients who present with metastatic disease at diagnosis.

The compound (+)-6-[amino(4-chlorophenyl)(1-methyl-1*H*-imidazol-5-yl)methyl]-4-(3-chloro-phenyl)-l-methyl-2(1H)-quinolinone, unless otherwise specified, is hereinafter referred to as the farnesyl transferase inhibitor according to the invention.

The pharmaceutically acceptable acid addition salts as mentioned hereinabove are meant to comprise the therapeutically active non-toxic acid addition salt forms which the farnesyl transferase inhibitor according to the invention is able to form. The compound can be converted in its pharmaceutically acceptable acid addition salts by treating said base form with an appropriate acid. Appropriate acids comprise, for example, inorganic acids such as hydrohalic acids, e.g. hydrochloric or hydrobromic acid; sulfuric; nitric; phosphoric and the like acids; or organic acids such as, for example, acetic, propanoic, hydroxyacetic, lactic, pyruvic, oxalic, malonic, succinic (*i.e*. butanedioic acid), maleic, fumaric, malic, tartaric, citric, methanesulfonic, ethanesulfonic, benzenesulfonic, *p*-toluenesulfonic, cyclamic, salicylic, *p*-aminosalicylic, pamoic and the like acids.

The terms acid addition salt also comprise the hydrates and the solvent addition forms which the farnesyl transferase inhibitor according to the invention is able to form. Examples of such forms are e.g. hydrates, alcoholates and the like.

The term stereochemically isomeric form of the farnesyl transferase inhibitor according to the invention defines all possible compounds made up of the same atoms bonded by the same sequence of bonds but having different three-dimensional structures which are not interchangeable, which the compound may possess. Unless otherwise mentioned or indicated, the chemical designation of a compound encompasses the mixture of all possible stereochemically isomeric forms which said compound may possess. Said mixture may contain all diastereomers and/or enantiomers of the basic molecular structure of said compound. All stereochemically isomeric forms of the farnesyl transferase inhibitor according to the invention, both in pure form or in admixture with each other are intended to be embraced within the scope of the present invention.

Whenever used hereinafter, the term "famesyl transferase inhibitor according to the invention" is meant to include also the pharmaceutically acceptable acid addition salts and all stereoisomeric forms.

The farnesyl transferase inhibitor according to the invention can be prepared and formulated into pharmaceutical compositions by methods known in the art; suitable examples can be found in WO-97/21701. To prepare the aforementioned pharmaceutical compositions, a therapeutically effective amount of the particular compound, optionally in addition salt form, as the active ingredient is combined in intimate admixture with a pharmaceutically acceptable carrier, which may take a wide variety of forms depending on the form of preparation desired for administration. These pharmaceutical compositions are desirably in unitary dosage form suitable, preferably, for systemic administration such as oral, percutaneous, or parenteral administration; or topical administration such as via inhalation, a nose spray, eye drops or via a cream, gel, shampoo or the like. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable solutions may be formulated in an oil for prolonged action. Appropriate oils for this purpose are, for example, peanut oil, sesame oil, cottonseed oil, corn oil, soy bean oil, synthetic glycerol esters of long chain fatty acids and mixtures of these and other oils. Injectable suspensions may also be prepared in which case appropriate liquid carriers, suspending agents and the like may be employed. In the compositions suitable for percutaneous administration, the carrier optionally comprises a penetration enhancing agent and/or a suitable wettable agent, optionally combined with suitable additives of any nature in minor proportions, which additives do not cause any significant deleterious effects on the skin. Said additives may facilitate the administration to the skin and/or may be helpful for preparing the desired compositions. These compositions may be administered in various ways, e.g., as a transdermal patch, as a spot-on or as an ointment. As appropriate compositions for topical application there may be cited all compositions usually employed for topically administering drugs e.g. creams, gellies, dressings, shampoos, tinctures, pastes, ointments, salves, powders and the like. Application of said compositions may be by aerosol, e.g. with a propellent such as nitrogen, carbon dioxide, a freon, or without a propellent such as a pump spray, drops, lotions, or a semisolid such as a thickened composition which can be applied by a swab. In particular, semisolid compositions such as salves, creams, gellies, ointments and the like will conveniently be used.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

Preferably, a therapeutically effective amount of the pharmaceutical composition comprising the farnesyl protein transferase inhibitor is administered orally or parenterally. Said therapeutically effective amount is the amount that effectively prevents growth or reduces the size of breast cancer tumors in patients. On the basis of the current data, it appears that a pharmaceutical composition comprising (+)-6-[amino(4-chlorophenyl) (1-methyl-1*H*-imidazol-5-yl)methyl]-4-(3-chlorophenyl)-1-methyl-2(1*H*)-quinolinone as the active ingredient can be administered orally in an amount of from 10 to 1500 mg daily, either as a single dose or subdivided into more than one dose. A preferred amount ranges from 100 to 1,000 mg daily. A particularly preferred dosage for such a compound is 300mg administered twice daily. This treatment can be given either continuously or intermittently in cycles of 3-4 weeks with treatment given for 1-21 days per cycle.

### Clinical Study

(+)-6-[amino(4-chlorophenyl) (1-methyl-1*H*-imidazol-5-yl)methyl]-4-(3-chlorophenyl)-1-methyl-2(1*H*)-quinolinone, referred to below as compound 75, was tested in patients with advanced breast cancer as described in the following account of a clinical study.

The clinical study involved 27 patients with advanced breast cancer. The median age of the patients was 59 years (range 35-80 years). Adjuvant chemo and/or endocrine therapy had been received by 15 and 16 patients respectively. Prior therapy for advanced disease included second-line hormonal therapy in 18 (67%) patients and/or one chemotherapy regimen only in 14 (52%) patients. Treatment was well tolerated, with myelosuppression the most frequent and dose-limiting toxicity. The first 6 patients treated with 400 mg of compound 75 twice daily developed grade 3/4 neutropenia after a median of 26 days; 5 were re-treated with dose reduction following neutrophil recovery without further haematological toxicity. The subsequent 21 patients received 300 mg of compound 75 twice daily; 6 (29%) developed grade 3/4 neutropenia after a median of 32 days, with one episode of fever. Neutrophil recovery occurred over 1-2 weeks in all cases. Thrombocytopenia (grade 3) occurred in 3 (11%) patients. Non-haematological toxicities included: grade 2/3 parasthesia/numbness in 7 (26%) patients occurring after a median of 10 weeks therapy; grade 2/3 diarrhoea in 3 (11%) patients; skin rash in 3 (11%); fatigue in 8 (28%). 26 patients were able to be evaluated for tumour response; 8 withdrew early (<12 weeks) due to either progression of disease and/or toxicity while 18 patients received at least 3 months treatment (range 12-36+ weeks). Tumour shrinkage of at least 50% in volume was seen in 3 (12%) patients, sites of response included liver, lung, lymph nodes and skin nodules. A further 9 (35%) patients had stable disease, i.e. no progression of tumour growth, at the 3 month evaluation. These results demonstrated that compound 75 has clinical activity in advanced breast cancer.

## Claims

1. Use of a farnesyl protein transferase inhibitor for the preparation of a pharmaceutical composition for treating advanced breast cancer, wherein said farnesyl protein transferase inhibitor is (+)-6-[amino(4-chlorophenyl)(1-methyl-1*H-*imidazol-5-yl)methyl]-4-(3-chloro-phenyl)-1-methyl-2(1*H*)-quinolinone; or a pharmaceutically acceptable acid addition salt or stereochemically isomeric form thereof.

2. The use as claimed in claim 1 wherein the pharmaceutical composition is for oral or parenteral administration.

3. The use as claimed in claim 1 or claim 2 wherein the pharmaceutical composition is in dosage unit form.

4. The use as claimed in any of the preceding claims wherein the pharmaceutical composition is for oral administration in an amount of the said farnesyl protein transferase inhibitor of from 10 to 1500 mg daily.

5. The use as claimed in claim 4 wherein the pharmaceutical is composition is for oral administration in an amount of the said farnesyl protein transferase inhibitor of from 100 to 1000 mg daily.

6. The use as claimed claim 5 wherein the pharmaceutical is composition is for oral administration in an amount of the said farnesyl protein transferase inhibitor of 300 mg twice daily.

## Revendications

1. Utilisation d'un inhibiteur de la farnésyl protéine transférase pour la préparation d'une composition pharmaceutique destinée au traitement du cancer du sein avancé, dans laquelle ledit inhibiteur de la farnésyl protéine transférase est la (+)-6-[amino(4-chlorophényl)(1-méthyl-1*H*-imidazol-5-yl)-méthyl]-4-(3-chlorophényl)-1-méthyl-2(1*H*)quinolinone ; ou un sel d'addition d'acide pharmaceutiquement acceptable ou une forme stéréochimiquement isomère de celle-ci.

2. Utilisation selon la revendication 1, dans laquelle la composition pharmaceutique est destinée à une administration par voie orale ou parentérale.

3. Utilisation selon la revendication 1 ou la revendication 2, dans laquelle la composition pharmaceutique est sous forme d'unité de dosage.

4. Utilisation selon l'une quelconque des revendications précédentes, dans laquelle la composition pharmaceutique est destinée à une administration par voie orale en une quantité dudit inhibiteur de la farnésyl protéine transférase allant de 10 à 1500 mg par jour.

5. Utilisation selon la revendication 4, dans laquelle la composition pharmaceutique est destinée à une administration par voie orale en une quantité dudit inhibiteur de la farnésyl protéine transférase allant de 100 à 1000 mg par jour.

6. Utilisation selon la revendication 5, dans laquelle la composition pharmaceutique est destinée à une administration par voie orale en une quantité dudit inhibiteur de la farnésyl protéine transférase de 300 mg deux fois par jour.

## Patentansprüche

1. Verwendung eines Farnesylproteintransferaseinhibitors zur Herstellung einer pharmazeutischen Zusammensetzung zur Behandlung von fortgeschrittenem Brustkrebs, wobei es sich bei dem Farnesylproteintransferaseinhibitor um (+)-6-[Amino-(4-chlorphenyl)(1-methyl-1*H*-imidazol-5-yl)methyl]-4-(3-chlorphenyl)-1-methyl-2(1*H*)-chinolinon oder ein pharmazeutisch unbedenkliches Säureadditionssalz oder eine stereochemisch isomere Form davon handelt.

2. Verwendung nach Anspruch 1, wobei die pharmazeutische Zusammensetzung zur oralen oder parenteralen Verabreichung bestimmt ist.

3. Verwendung nach Anspruch 1 oder 2, wobei die pharmazeutische Zusammensetzung in Einheitsdosisform vorliegt.

4. Verwendung nach einem der vorhergehenden Ansprüche, wobei die pharmazeutische Zusammensetzung zur oralen Verabreichung in einer Menge an Farnesylproteintransferaseinhibitor von täglich 10 bis 1.500 mg bestimmt ist.

5. Verwendung nach Anspruch 4, wobei die pharmazeutische Zusammensetzung zur oralen Verabreichung in einer Menge an Farnesylproteintransferaseinhibitor von täglich 100 bis 1.000 mg bestimmt ist.

6. Verwendung nach Anspruch 5, wobei die pharmazeutische Zusammensetzung zur oralen Verabreichung in einer Menge an Farnesylproteintransferaseinhibitor von zweimal täglich 300 mg bestimmt ist.
